# EUROPEAN PATENT APPLICATION

(11) **EP 4 847 604 A1**
(43) Date of publication of application: **02.12.2026**
(21) Application number: 25382546.7
(22) Date of filing: 29.05.2025
(51) Int. Cl.: C12N 1/20, A61K 35/74, A61Q 19/00, C12P 19/14

(54) **BACTERIAL STRAIN OF PSEUDOALTEROMONAS SP., EXTRACELLULAR PRODUCTS THEREOF AND THEIR USES**

(71) Applicant: Fundación AZTI - AZTI Fundazioa, 48395 Sukarrieta (Bizkaia) (ES)
(72) Inventor: PÉREZ CRUZ, Carla, Sukarrieta (ES); Alonso Sáez, Laura, Sukarrieta (ES); Gómez Férnandez, Paloma, Sukarrieta (ES); Mentxaka Miranda, Gartze, Sukarrieta (ES); Garcia Salvador, Adrian, Sukarrieta (ES); Goñi de Cerio, Felipe, Sukarrieta (ES); Santos Vizcaíno, Edorta, Sukarrieta (ES); Igartua Olaechea, Manuela, Sukarrieta (ES); Las Heras Zapata, Kevin, Sukarrieta (ES); Hernandez Martin, Rosa Maria, Sukarrieta (ES); Sanmartin Sierra, Esther, Sukarrieta (ES); Estupiñán Romero, Mónica, Sukarrieta (ES)
(74) Representative: Pons IP

(57) **Abstract**

The present invention relates to a novel bacterial strain *Pseudoalteromonas* sp. with deposit number CECT 30790, referred as to the strain of the invention. The invention also relates to a composition that comprises an EPS mainly composed of galacturonic acid, N-acetylgalactosamine, mannose, N-acetylglucosamine, and in a lower amount, galactose, a mixture of other acetylated deoxy amino sugars, glucose, and other monosaccharides like heptoses. The invention also relates to therapeutic and cosmetic uses of the aforementioned composition.

## Description

The present invention relates to a marine bacterium, particularly an exopolysaccharide-producing marine bacterium. The present invention also relates to the extracellular products produced by the marine bacterium of the invention, as well as pharmaceutical, nutraceutical, cosmetic or food compositions comprising it and uses thereof. Thus, the present invention is within the biotechnology field.

### BACKGROUND ART

In nature, marine bacteria are embedded in a complex polymeric extracellular matrix (ECM), which they secrete as a growth and/or adaptation strategy to the environment. Specifically, the ECM forms a layer surrounding the cell, providing effective protection against environmental changes through, for example, prevention of desiccation, protection against toxic stress and barrier against potential predators (Nwodo, et al., 2012, International Journal of Molecular Sciences, 13, 14002-14015.). ECM production is also among the physiological strategies used by microorganisms present in hypersaline environments to survive in conditions of high salinity. These organisms are capable of producing significant amounts of ECM with high surface activity and/or with rheological properties interesting for applications in the food, cosmetic and pharmaceutical sectors (Poli, et al., 2010, Marine Drugs, 8, 1779-1802).

ECM is mainly composed of exopolysaccharides (EPS), although they are also formed by proteins, nucleic acids and membrane vesicles (MVs) (Flemming and Wingender, 2010, Nat Rev Microbiol, 8, 623-633). The EPS produced by marine bacteria are usually heteropolysaccharides with a size greater than 100,000 kDa, formed by oligosaccharide repeats composed of 3 or 4 different monosaccharides, which can be hexose, pentose, uronic acids or amino sugars. In addition, organic or inorganic substituents, namely sulfate, phosphate, acetic acid, succinic acid and pyruvic acid may also be present (Poli, et al., 2010, Marine Drugs, 8, 1779-1802). At the biotechnological level, EPS are very promising since they contain in their composition rare sugars. The scarcity of these sugars in nature makes them very valuable since they can be used as building blocks for the synthesis of nucleoside analogues applied as viral agents and in fine chemistry (Roca et al., 2015, Front Microbiol 6, 288). The composition and structure of EPS can vary greatly according to the type of microorganisms, even becoming species-specific (Decho, 1990, Oceanogr Mar Biol Ann Rev 28: 73-153).

The MVs present in the ECM are spherical structures, with a diameter between 20-300 nm that derive from the bacterial envelope and encapsulate bioactive substances such as quorum molecules or enzymes (Kulp and Kuehn. "Biological functions and biogenesis of secreted bacterial outer membrane vesicles", 2010, Annual Review Microbiol, 64, 163-84). One of the main components of MVs are glycolipids. These are amphyphilic substances formed by a hydrophilic region composed of di- or polysaccharides and a hydrophobic region formed by fatty acids. It has been proposed that glycolipids produced by microorganisms have interesting functionalities for the pharmaceutical, cosmetic and food industry since they can alter the permeability and hydrophobicity of the cell membrane, have immunomodulatory, antibacterial or antiviral activity and exhibit moisturizing and foaming properties. In addition, they have low toxicity and are biodegradable (Mnif and Ghribi, 2015, Carbohydrate Research, 416, 59-69).

In general, bacteria of the genus *Pseudoalteromonas* isolated from cold environments secrete abundant MVs into their matrix (Frias et al., 2010, Microbial Ecology, 59, 476-86). Marine bacterial strains belonging to this genus also stand out for their ability to produce EPS with different compositions:
*Pseudoalteromonas agarivorans* produces an EPS formed mainly by glucose (90.28%) and presents a lower proportion of mannose (6.60%) (Hao. et al., 2019, Marine drugs, 17, 703).
*Pseudoalteromonas sp. SM9913,* isolated from marine sediments, secretes an acetylated EPS consisting mainly of glucose (73.0%) and arabinose (11.0%) (Qin G. et al, 2007, Microbiology, 153, 1566-1572).
*Pseudoalteromonas* sp. YU16-DR3A secretes an EPS composed of 69% carbohydrates and 7.6% proteins with a molecular weight of 20 kDa. Monosaccharide analysis showed the presence of fucose, erythrose, ribose, and glucose as monomers (Dhanya et al., 2022, International Microbiology, 25(2), 285-295).

WO2012072245A2 refers to an EPS isolated from *Pseudoalteromonas* sp. with a composition by weight of 3% to 12% mannose, 12% to 34% glucose, 12% to 34% glucuronic acid, 2% to 20% galacturonic acid, 12% to 34% galactose and 2% to 18% N-acetylglucosamine. This invention relates to a cosmetic or dermopharmaceutical composition for the treatment and/or care of skin, mucous membranes, hair and/or nails comprising an effective amount of EPS and at least one excipient, adjuvant. It has a rejuvenating and moisturizing effect. Adjuvants include, emulsifiers, healing, inhibitors of collagen and elastin degradation, anti-inflammatories, cell proliferation stimulants.

WO2016066857A1 discloses an extract of molecular weight less than 10,000 Da produced by a strain of the species *Pseudoalteromonas antarctica* NF3 for use in the treatment of skin and/or mucous membranes, in the prevention of inflammation, skin cancer, acne, seborrhea, seborrheic dermatitis, hidradenitis suppurativa or as a skin photoprotectant. It inhibits the MC5R receptor and/or stimulates collagen synthesis.

WO2010043346A1 describes a cosmetic or dermopharmaceutical composition containing ferment extract of *Pseudoalteromonas antarctica* NF3 for the treatment and/or care of conditions, disorders and/or pathologies associated with an alteration of the barrier function of the skin, scalp and/or nails, and preferably an alteration of keratinization.

Therefore, due to the growing interest in the field, there is a need in the prior art to provide new extracellular exopolysaccharides that can be used in the cosmetic, pharmaceutical, or food industry.

### DESCRIPTION OF THE INVENTION

The present invention discloses a novel bacterial strain belonging to the genus *Pseudoalteromonas* which is able to produce extracellular products having properties of interest to the food industry, biomedical and cosmetic fields.

The bacterium disclosed in the present invention is *Pseudoalteromonas sp.* with the deposit number CECT 30790, a marine bacterium isolated from the seawater of the Bay of Biscay in Spain. The strain *Pseudoalteromonas sp.* with the deposit number CECT 30790 was received on 24^{th} February 2023, for patent deposit according to the Budapest Treaty, at the "Colección Española de Cultivos Tipo, CECT" (Spanish Type Culture Collection) with the address: Parc Cientific de la Universitat de València, Carrer del Catedràtic Agustin Escardino Benlloch, 9, 46980 Paterna, Valencia, Spain; the depositor being Fundación AZTI-AZTI Fundazioa with the address Txatxarramendi ugartea s/n, 48395 Sukarrieta (Vizcaya), Spain.

In one aspect, the invention relates to a bacterial strain *Pseudoalteromonas* sp. with deposit number CECT 30790, hereinafter "the strain of the invention".

In the context of the present invention, the strain of the invention can be present as the wild-type strain of the invention or as the mutant strain of the invention. The term wild-type refers to a strain having a phenotype, genotype, or gene that predominates in a natural population of organisms. The term "mutant" or "derived" refers to the strain of the invention comprising at least one variation or mutation in its genome, wherein the variation in the genome of the derived or mutant strain of the invention is not present in the genome of the wild-type strain of the invention. The variation in the genome may be a point mutation, an insertion, a deletion, or combinations thereof, wherein the variation or variations in the genome of the mutant strain of the invention do not affect the cell viability. Thus, the strain of the invention can be as the wild-type strain of the invention in the form of a living or inactivated cell or as the mutant strain of the invention in the form of a living or inactivated cell.

After analysing the extracellular products released by the strain of the invention, the inventors determined that said strain produces a novel exopolysaccharide with a particular monosaccharide composition mainly composed of galacturonic acid, *N-*acetylgalactosamine, mannose and *N*-acetylglucosamine. In a lower amount, the exopolysaccharide produced by the strain of the invention also comprises galactose, a mixture of acetylated desoxyamino monosaccharides (other desoxyamino monosaccharides different form *N*-acetylglucosamine or *N*-acetylglucosamine), heptoses, and glucose.

Therefore, in another aspect, the invention relates to a composition comprising an exopolysaccharide (EPS), hereinafter "the composition of the invention", that comprises galacturonic acid, *N*-acetylgalactosamine, mannose, *N-*acetylglucosamine, galactose and glucose.

As used in the present invention, the terms "EPS" or "exopolysaccharide" should be understood to mean high molecular weight polymers (usually >100 kDa) that are composed of sugar residues (monosaccharides) and released by bacteria. So, in a preferred embodiment, the EPS of the invention has a molecular weight greater than 100 kDa. As the skilled person knows, marine bacterial exopolysaccharides are usually composed of different monosaccharide components linked forming an heteropolysaccharide. According to the monosaccharide composition determined by the inventors, the exopolysaccharide of the composition of the invention is an heteropolysaccharide.

The present inventors also found that the extracellular products produced by the strain of the invention do not comprise glucuronic acid in appreciable amounts.

Thus, in a preferred embodiment, the exopolysaccharide, and therefore, the composition of the invention does not comprise glucuronic acid in its monosaccharide composition.

Surprisingly, the extracellular products produced by the strain of the invention further comprises other N-acetylated deoxy amino sugars.

Thus, in another preferred embodiment of the composition of the invention, the exopolysaccharide further comprises *N*-acetylated deoxy amino sugars that are not *N*-acetylglucosamine nor *N*-acetylgalactosamine.

After the analysis of the extracellular products produced by the strain of the invention, it was determined that it also comprised heptoses.

Therefore, in another preferred embodiment of the composition of the invention, the exopolysaccharide further comprises at least one heptose.

In another preferred embodiment of the composition of the invention, the exopolysaccharide comprises galacturonic acid, N*-*acetylgalactosamine, mannose, N-acetylglucosamine, galactose, glucose, *N*-acetylated deoxy amino sugars that are not *N*-acetylglucosamine nor *N*-acetylgalactosamine and at least one heptose.

Moreover, the extracellular products produced by the strain of the invention contain a lower amount of glucose compared to the prior art.

In another preferred embodiment of the composition of the invention, the exopolysaccharide comprises less than 5 % by weight of glucose respect to the total weight of the composition.

In a more preferred embodiment, the composition of the invention comprises an exopolysaccharide, wherein said exopolysaccharide comprises from 10 to 20 % by weight of galacturonic acid, from 5% to 12% by weight of *N*-acetylgalactosamine, from 2 % to 8 % by weight of *N*-acetylglucosamine, from 4 to 7% by weight of mannose, from 2 % to 5% by weight of galactose and 1% to 4% by weight of glucose, wherein the percentages are expressed with respect to the total weight of the composition.

In a more preferred embodiment of the composition of the invention, the exopolysaccharide further comprises from 1% to 8% by weight of *N*-acetylated deoxy amino sugars that are not *N*-acetylglucosamine nor *N*-acetylgalactosamine, wherein the percentages are expressed with respect to the total weight of the composition of invention.

In the present invention the term "amino sugar" refers to a monosaccharide in which a hydroxyl group has been replaced with an amine group. The term "deoxy amino sugars" is an amino sugar that has an hydroxyl group replaced with a hydrogen atom. The term "*N*-acetyl amino sugar" is an amino sugar wherein a hydrogen of the amine group is substituted by an acetyl group.

In another preferred embodiment of the composition of the invention, the exopolysaccharide further comprises from 1% to 6% by weight of at least one heptose, wherein the percentages are expressed with respect to the total weight of the composition of the invention. The term "heptose" as used herein, refers to a monosaccharide with seven carbon atoms.

In a more preferred embodiment of the composition of the invention, the exopolysaccharide comprises:
- from 10% to 20 % by weight of galacturonic acid;
- from 5 % to 12% by weight of N-acetylgalactosamine;
- from 2 % to 8% by weight of N-acetylglucosamine;
- from 4 % to 7% by weight of mannose;
- from 2% to 5% by weight of galactose;
- from 1 to 4 % by weight of glucose;
- from 1 to 8% by weight of *N*-acetylated deoxy amino sugars which are not *N-*acetylglucosamine nor *N*-acetylgalactosamine, and/or
- from 1% to 6% by weight of at least one heptose,
wherein the percentages are expressed with respect to the total weight of the composition of the invention.

In a more preferred embodiment of the composition of the invention, the exopolysaccharide comprises:
- from 13% to 18 %, more preferably from 14 to 17%, by weight of galacturonic acid;
- from 7 % to 11%, more preferably from 7 to 9%, by weight of *N-*acetylgalactosamine;
- from 3 % to 6%, more preferably from 4 to 5%, by weight of *N-*acetylglucosamine;
- from 4 % to 6% by weight of mannose, more preferably 5% by weight of mannose;
- from 2% to 4% by weight of galactose, more preferably 3% by weight of galactose;
- from 1 to 2 % by weight of glucose;
- from 1 to 7%, more preferably from 1 to 6%, by weight of *N*-acetylated deoxy amino sugars which are not *N*-acetylglucosamine nor *N-*acetylgalactosamine, and/or
- from 1% to 6%, more preferably from 1 to 4%, by weight of at least one heptose,
wherein the percentages are expressed with respect to the total weight of the composition of the invention.

The monosaccharide composition of the EPS of the composition of the invention is determined by gas chromatography-mass spectrometry using the method based on the formation of per-O-acetyl methyl glycosides (C. de Castro et al., Methods Enzymology 480 (2010) 89-115). More preferably, the monosaccharide composition of the EPS of the composition of the invention is determined as in the Example 4 of the present document.

In another preferred embodiment, the EPS of the composition of the invention is produced by the strain of the invention.

EPSs may be released into the extracellular space together with other biomolecules such as lipids, proteins, nucleic acids and/or extracellular vesicles.

According to electronic microscopy imaging, the present inventors found that the EPS is released conforming an amorphous network comprised in the extracellular matrix of the strain of the invention, wherein said extracellular matrix also comprises extracellular vesicles.

Thus, in another preferred embodiment, the composition of the invention further comprises extracellular vesicles.

The terms "extracellular vesicles", "EVs", "membrane vesicles" and "MVs" interchangeably used herein, refer to lipid bilayer-delimited particles released from cells, such as the strain of the invention. The lipid bilayer delimits a core and may comprise embedded proteins and other elements such as carbohydrates. The core is a hydrophilic environment that may comprise proteins, DNA and/or RNA.

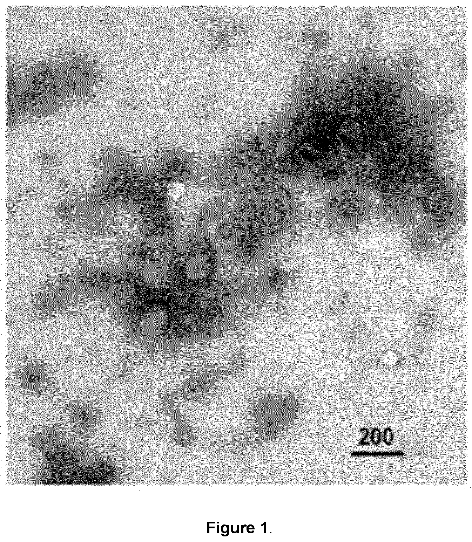

In a preferred embodiment of the composition of the invention, the extracellular vesicles have a diameter between 20 nm and 200 nm as determined by transmission electronic microscopy (Figure 1).

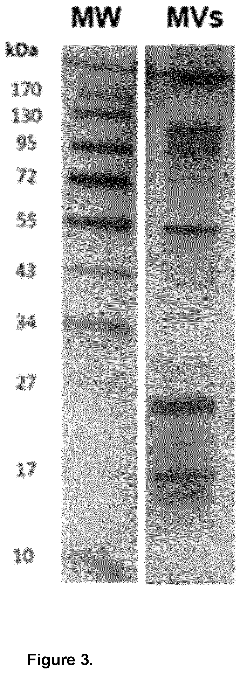

According to the assays performed by the inventors, the extracellular products released by the strain of the invention also comprise proteins with different molecular weights as determined by SDS-PAGE technique (Figure 3).

Thus, in a preferred embodiment, the composition of the invention further comprises proteins.

In a preferred embodiment of the composition of the invention, the proteins have a molecular weight between 15 kDa and 130 kDa.

In a more preferred embodiment of the composition of the invention, proteins comprise four fractions of different molecular weights:
- protein fraction of molecular weight between 95 kDa and 130 kDa;
- protein fraction of molecular weight between 50 kDa and 60 kDa;
- protein fraction of molecular weight between 25 kDa and 35 kDa; and
- protein fraction of molecular weight between 15kDa and 25 kDa.

In a preferred embodiment, the composition of the invention comprises a from 3.10% to 3.5% by weight of proteins with respect to the total weight of the composition.

The present inventor also determined that the extracellular products produced by the strain of the invention also comprises DNA. As used herein, the term "DNA" refers to deoxyribonucleic acid.

Thus, in a preferred embodiment, the composition of the invention further comprises DNA.

In a more preferred embodiment of the composition of the invention, the concentration by weight of DNA is from 0.45% to 0.70 %, with respect to the total weight of the composition.

The amount of proteins may be determined by methods known for the skilled person in the art, such as, without limitation, fluorometric methods (e.g. UV absorption or Qubit Protein assay), colorimetric methods (e.g. Bradford, Coomassie, Lowry or Bicinchoninic acid method) or densitometry methods (e.g. gel electrophoresis); preferably by a fluorometric method.

The amount of DNA may be determined by methods known for the skilled person in the art, such as, without limitation, fluorometric methods (e.g. UV absorption or Qubit DNA assay), densitometry methods (e.g. gel electrophoresis) or polymerase chain reaction assays (e.g. PCR, real time PCR assay); preferably by a fluorometric method.

In a preferred embodiment, the composition of the invention is an extracellular matrix; preferably a bacterial extracellular matrix.

As used herein, the terms "extracellular matrix" or "ECM" refers to a complex of mainly polymeric substances (exopolysaccharide) secreted by a cell (e.g., the strain of the invention), wherein ECM surrounds the surface of cell that secretes it. The main polymeric substance that conforms an ECM, specifically bacterial ECM, are exopolysaccharides. However, ECM also include other components such as, but not limited to, DNA, proteins, glycopeptides, lipids, nucleic acids and/or extracellular vesicles. ECMs are not solid, thus, preferably, in the present invention the ECM is liquid.

In a more preferred embodiment, the composition of the invention is an extracellular matrix, more preferably a bacterial extracellular matrix, and more preferably an extracellular matrix produced by the strain of the invention.

Thus, in another preferred embodiment, the proteins of the composition of the invention are ECM-associated proteins.

The term "ECM-associated proteins" refers to the proteins that are secreted together with the EPS and/or the extracellular vesicles in the composition of the invention (an extracellular matrix). Therefore, the ECM-associated proteins may be also understood as EPS-associated proteins and/or EV-associated proteins.

The exopolysaccharide, and/or the ECM comprising thereof, produced by the strain of the invention, may also be referred to as the extracellular products produced by the strain of the invention.

In some preferred embodiments, the composition of the invention is an isolated composition. The term "isolated" should be considered to mean material removed from its original environment in which it naturally occurs, for example, a bacterial strain isolated from the seawater. In the context of the present invention, the "isolated composition of the invention" (preferably, extracellular matrix) means that the composition of the invention is removed from the strain of the invention. As the skilled person knows, ECM may be removed from its original environment in which it naturally occurs by different methods such as, without limitation: alcohol precipitation, filtration, ultrafiltration, centrifugation or dialysis.

In another aspect, the invention relates to a pharmaceutical, nutraceutical, cosmetic or food composition (hereinafter the "pharmaceutical, nutraceutical, cosmetic or food composition of the invention) that comprises the composition of the invention.

As used herein, the term "pharmaceutical composition" refers to a composition or mixture that comprises an active agent (e.g., the composition of the invention) wherein the active agent produces a physiological result or modulates a biological process when administered to a subject, e.g., health benefits. Active agents are distinguishable from other components of the delivery compositions, such as carriers, diluents, binders, colorants, etc.

As used herein, the term "nutraceutical composition" refers to a composition or mixture that comprises nutrients and active agent. Therefore, the nutraceutical composition provides nutritional benefits to the subject to which it is administered, by the provision of essential nutrients like proteins, carbohydrates, lipids, vitamins or minerals. In addition, the active agent comprised in the nutraceutical composition (e.g. the composition of the invention) provides to the subject a health benefit.

The term "cosmetic composition" refers to any substance or mixture intended to be placed in contact with the various external parts of the body of a subject (epidermis, hair system, nails, lips and external genital organs) or with the teeth and the mucous membranes of the oral cavity with a view exclusively or mainly to cleaning them, perfuming them, changing their appearance and/or correcting body odours and/or protecting them or keeping them in good condition. The subject is preferably human.

As used herein the term "subject", refers to a human being or a non-human animal, where examples of non-human animal include, without limitation, dogs, cats, cows, pigs, goats, sheep, fish, reptiles, non-human primates, mice or rats.

In a preferred embodiment, the pharmaceutical, nutraceutical, cosmetic or food composition of the invention further comprises an excipient and/or carrier, wherein the excipient/or carrier is a pharmaceutically acceptable excipient and/or carrier when the composition is a "pharmaceutical composition"; the excipient and/or carrier is a nutraceutically acceptable excipient and/or carrier when the composition is a "nutraceutical composition"; and wherein the excipient or carrier is an cosmetically acceptable excipient and/or carrier when the composition is a "cosmetic composition".

The term "excipient" refers to a substance that aids in the absorption of any of the components of the composition of the present invention, stabilizes said components, or aids in the preparation of the composition Thus, the excipients could have the function of keeping the components together, such as starches, sugars or celluloses; sweeten function; coloring function; protective function of the active agent, such as to isolate it from air and/or moisture; function filler of a tablet, capsule, pill or any other form of presentation such as, for example, dibasic calcium phosphate; disintegrating function to facilitate the removal of components; without eliminating other types of excipients not mentioned in this paragraph.

The "carrier" is preferably an inert substance. Carrier functions are to facilitate the incorporation of other components or compounds, allow better dosage and administration and/or give consistency and form to composition. Therefore, the carrier is a substance used in the drug to dilute any of the components or compounds of the composition of the present invention to a given volume or weight; or that even without diluting these components or compounds, it is able to allow better dosage and administration and/or give consistency. The carrier can be natural or unnatural. Examples of carriers include, without being limited thereto, water, salt solutions, alcohol, vegetable oils, polyethylene glycols, gelatine, lactose, starch, amylose, magnesium stearate, talc, surfactants, silicic acid, viscous paraffin, perfume oil, monoglycerides and diglycerides of fatty acids, fatty acid esters petroetrals, hydroxymethylcellulose, polyvinylpyrrolidone and the like.

The terms "food composition" or "food product", used interchangeably, encompass any composition containing nutrients, whether for human or animal consumption, whether comprised of a single ingredient or a mixture of ingredients, whether liquid, liquid containing or solid, whether primarily carbohydrate, fat, protein or any mixture thereof, whether edible per se or requiring processing like cooking, mixing, cooling, mechanical treatment and the like. More specifically, the term food composition may encompass, without limitation, functional food compositions, dietary supplements, animal feed, animal feed supplements and food for special nutritional purposes. Nonlimiting examples of food compositions include edible beverage (e.g. juices, wines, milk based beverages, etc), dairy products (e.g. dairy fermented products such as yoghurts), concentrates, jams, jellies, preserves or pastes.

The pharmaceutical, nutraceutical, cosmetic or food composition comprising the composition of the invention may be present in a form of, for example, tablets, capsules, pills, powders granulates, suspensions, emulsions, solutions, gels (including hydrogels), pastes, ointments, creams, plasters, drenches, delivery devices, suppositories, enemas, injectables, implants, sprays, aerosols or other suitable forms.

In some preferred embodiments, the composition of the invention is comprised in a pharmaceutical composition present in a form suitable for parenteral administration, mucosal administration, intravenous administration, subcutaneous administration, topical administration, intradermal administration, oral administration, sublingual administration, intranasal administration, or intramuscular administration.

In some preferred embodiments, the composition of the invention is comprised in a cosmetic composition present in a form suitable for mucosal administration, subcutaneous administration, topical administration, intradermal administration, oral administration, or sublingual administration.

In some preferred embodiments, the composition of the invention is comprised in a nutraceutical or a food composition present in a dosage form suitable for oral administration or sublingual administration.

In a preferred embodiment, the pharmaceutical, nutraceutical, cosmetic or food composition comprises the composition of the invention at an amount with regard to the total weight of the pharmaceutical, nutraceutical, cosmetic or food composition, between 0.0001% (in weight) and 20% (in weight); preferably between 0.0001% (in weight) and 10% (in weight); more preferably between 0.0001% (in weight) and 5% (in weight).

Thus, in another aspect, the invention relates to a method for the obtention of a composition that comprises an exopolysaccharide, hereinafter "the obtention method of the invention", comprising the following steps: a) culturing bacterial strain of the invention; and b) isolating the composition comprising the EPS from the secreted fraction of the culture.

In a preferred embodiment, the composition that comprises an exopolysaccharide produced by the obtention method of the invention, is the composition of the invention; preferably the composition obtained by the obtention method of the invention being the extracellular matrix produced by the strain of the invention.

Therefore, in another preferred embodiment, the composition of the invention is obtainable by the obtention method of the invention. Alternatively, the composition of the invention is produced by the strain of the invention.

In the present invention, the term "secreted fraction of the culture" refers to all the components released to the outside of the bacteria that are cultured, referring not only to the components that are released into the culture medium but also the components that are adhered to the outer surfaces of the bacterial cells.

In a preferred embodiment of the obtention method of the invention, the bacterial strain of the invention is cultured in a suitable culture medium at a temperature ranging from 5°C to 30°C; preferably at 10°C.

The term "suitable culture medium" refers to a culture medium that contains all the minimal nutritional elements that allow the growth and/or survival of the bacteria that are present in it.

Examples of suitable culture mediums include, without limitation; MM10, Zobell broth, or Marine Broth 2216 with or without glucose. In a preferred embodiment the culture medium of the step a) of the obtention method of the invention, is MM10 medium with a composition of salts per liter: Na₂HPO₄ (5 g), KH₂PO₄ (2 g), NaCl (30 g), NH₄Cl (7 g), MgSO₄x 7 H₂0 (0.018 g ), CaCl₂ (0.05 g), glucose (20 g), peptone (5 g) and yeast extract (1 g).

In another preferred embodiment of the obtention method of the invention, the incubation time of step a) is at least for 2 hours. In a more preferred embodiment, the incubation time of step a) is between 1 day and 7 days. In a more preferred embodiment, the incubation time of step a) is 3 days.

In the present invention, the isolation in the step b) of the obtention method of the invention may be carried out by methods known in the art, such as, without limitation: alcohol precipitation, filtration, ultrafiltration, centrifugation, dialysis, or any combination thereof.

So, in a preferred embodiment of the obtention method of the invention, the isolation of the step b) is carried out by a method selected from the list consisting of alcohol precipitation, filtration, ultrafiltration, centrifugation, dialysis or any combination thereof; preferably ultrafiltration followed by dialysis.

In a more preferred embodiment of the obtention method of the invention, the isolation in the step b) of the obtention method of the invention is carried out by filtration followed by dialysis process, concentration and optionally followed by a further filtration. In a preferred embodiment the filtration is carried out with a membrane with a pore size between 0.15 µm and 0.50 µm. In a preferred embodiment, the dialysis is carried out with a membrane which retains molecules of a molecular weight greater than 10 kDa.

Surprisingly, the inventors found that the composition of the invention showed higher emulsification activity against different food or cosmetics oils and n-hexadecane, than other bacterial EPS or plant gums, namely xanthan gum or arabic gum, common ingredients used in the alimentary and cosmetic industry (Figures 4 and 5A). Moreover, the EPS demonstrated a better performance in producing stable emulsions compared to arabic gum (Figures 5B and 6) against different food or cosmetics oils and n-hexadecane.

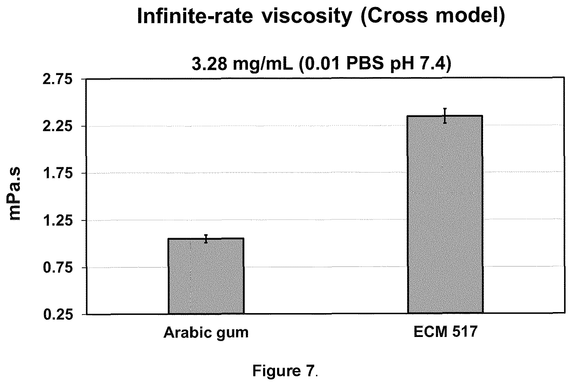

Additionally, the composition of the invention generated solutions with higher viscosity compared to arabic gum (Figure 7).

So, in another aspect, the invention refers to the use of the composition of the invention, as emulsifying, thickening and/or stabilizing agent.

The terms "emulsifying agent" or "emulsifiyer", used interchangeably, refer to a substance that helps products or compositions containing immiscible substances or components, like oil and water, to combine. The terms "thickening agent or thickener", interchangeably used in the present invention, refer to a substance which can increase the viscosity of a solution or a mixture without substantially changing its other properties. The terms "stabilizing agent" or "stabilizer", interchangeably used in the present invention, refer to a substance that, when added to a composition, maintain or enhance their original texture, physical and chemical characteristics. The use of composition of the invention as emulsifying, thickening and/or stabilizing agent may be directed to the manufacture of pharmaceutical, cosmetic, alimentary or food compositions, so the composition of the invention is added when manufacturing a pharmaceutical, cosmetic, alimentary or food compositions.

In another aspect, the present invention refers to the use of the composition of the invention as food ingredient for the manufacture of food products or compositions, wherein the composition of the invention is added when manufacturing food products or compositions.

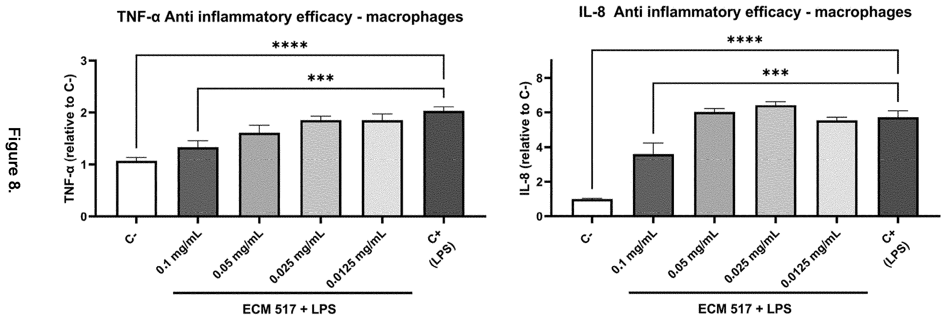

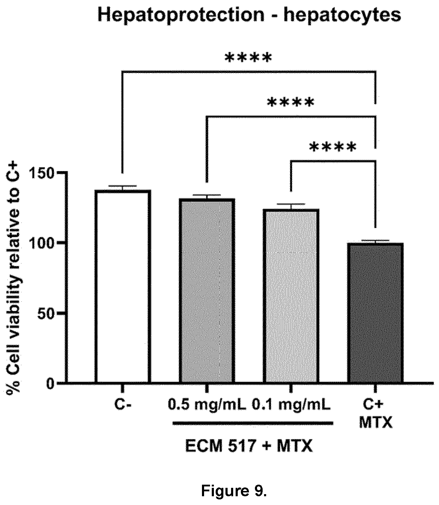

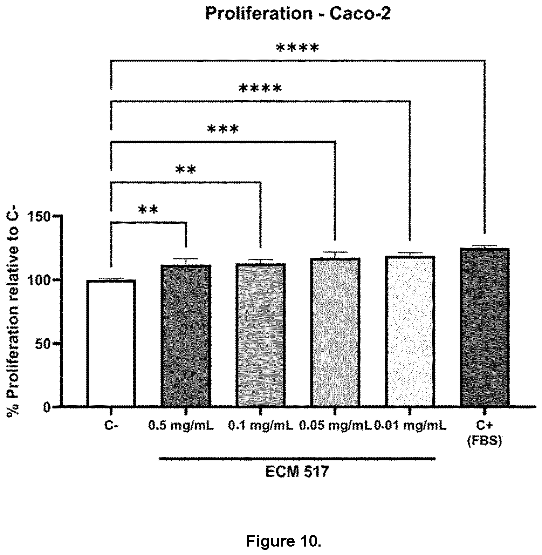

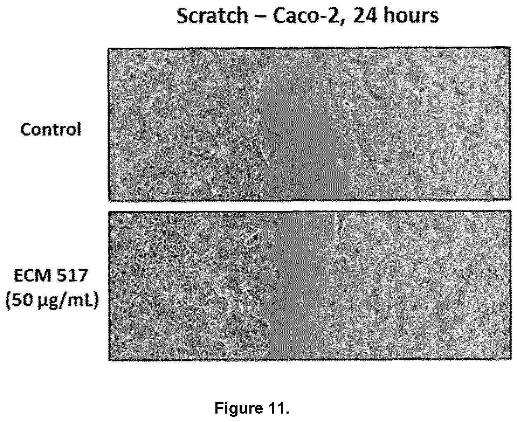

As the inventors also found, the extracellular products secreted by the strain of the invention, surprisingly showed anti-inflammatory properties (Figure 8) hepatoprotective properties in hepatocytes that were exposed to a hepatotoxic drug, (Figure 9), and it was also able to promote the proliferation of intestinal cells *in vitro* (Figure 10 and Figure 11).

Thus, in another aspect, the invention refers to the composition of the invention, the pharmaceutical composition or the nutraceutical composition comprising the composition of the invention for use as a medicament.

In another aspect, the invention relates to the composition of the invention, the pharmaceutical composition or the nutraceutical composition comprising the composition of the invention, for use in hepatoprotection, in the treatment and/or prevention of a hepatic disease, or in the treatment and/or prevention of a disease or condition associated with inflammation in a subject, hereinafter "the therapeutic use of the invention"; preferably wherein the hepatic disease is selected from the group consisting of liver transplantation, alcoholic or non-alcoholic fatty liver, hepatic fibrosis, hepatocirrhosis and hepatitis caused by virus or drug, alcoholic acute/chronic hepatic disease, fatty acid-induced fatty liver or acute/chronic hepatic disease derived from fatty liver or a hepatic disease is mediated by reactive oxygen species (ROS) or heavy metal; and preferably wherein the disease or condition associated with inflammation is selected from the list consisting, allergy, cardiovascular disease, neurologic disease, pulmonary disease, cancer, metabolic disease, auto-immune disease, gastrointestinal disease, and skin or mucosal disease or disorder.

In the present invention, the term "hepatoprotection" refers to the prevention of damage to the liver, being the opposite to hepatotoxicity.

In a preferred embodiment of the therapeutic use of the invention, the cardiovascular disease is arteriosclerosis.

In a preferred embodiment of the therapeutic use of the invention, the neurological disease is Alzheimer's disease.

In a preferred embodiment of the therapeutic use of the invention, the metabolic disease is diabetes mellitus.

In a preferred embodiment of the therapeutic use of the invention, the autoimmune disease is selected from the list consisting of Chron disease, arthritis and lupus erythematosus.

In a preferred embodiment of the therapeutic use of the invention, the pulmonary disease is asthma.

In a preferred embodiment of the therapeutic use of the invention, the cancer is skin cancer.

In a preferred embodiment of the therapeutic use of the invention, the gastrointestinal disease is selected from the list consisting of intestinal inflammatory disease, stomach ulcers, duodenal ulcers and irritable bowel syndrome.

In a preferred embodiment of the therapeutic use of the invention, the skin or mucosal disease or condition is selected from the list consisting of skin infection, dry skin, xerosis, hyperkeratosis, reactive hyperkeratosis, palmar and plantar hyperkeratosis, corns and calluses, actinic keratosis, non-actinic keratosis, atopic dermatitis, contact eczema, seborrheic dermatitis, dandruff, cradle cap on babies, acne, rosacea, nevus, ichthyosis, psoriasis, parakeratosis, pityriasis, lichen planus, palmoplantar keratoderma.

The terms "treat," "treating" or "treatment," and other grammatical equivalents as used throughout the present description, include alleviating, inhibiting or reducing symptoms, reducing or inhibiting severity of, reducing incidence of, prophylactic treatment of, reducing or inhibiting recurrence of, preventing, delaying onset of, delaying recurrence of, abating or ameliorating a disease or condition symptoms, ameliorating the underlying metabolic causes of symptoms, inhibiting the disease or condition, e.g., arresting the development of the disease or condition, relieving the disease or condition, causing regression of the disease or condition, relieving a condition caused by the disease or condition, or stopping the symptoms of the disease or condition.

The terms "prevent," "preventing" or "prevention," and other grammatical equivalents as used throughout the present description, include preventing additional symptoms, preventing the underlying metabolic causes of symptoms, inhibiting the disease or condition, e.g., arresting the development of the disease or condition and are intended to include prophylaxis. The terms further include achieving a prophylactic benefit. For prophylactic benefit, the compositions are optionally administered to an individual at risk of developing a particular disease, to an individual reporting one or more of the physiological symptoms of a disease, or to an individual at risk of reoccurrence of the disease.

In a preferred embodiment of the therapeutic use of the invention, the composition of the invention is administered, preferably comprised in a pharmaceutical composition or a nutraceutical composition, at a therapeutically effective amount. As used herein, the term "therapeutically effective amount" refers to any amount of a compound, agent, mixture or composition that is sufficient to fulfil its therapeutic intended purpose (e.g. hepatoprotection, treatment and/or prevention of a hepatic disease or a disease associated with inflammation).

In a more preferred embodiment of the therapeutic use of the invention, the therapeutically effective amount of the composition of the invention with regard to the total weight of the pharmaceutical or nutraceutical composition, is between 0.0001% (in weight) and 20% (in weight); preferably between 0.0001% (in weight) and 10% (in weight); more preferably between 0.0001% (in weight) and 5% (in weight).

In another preferred embodiment of the therapeutic use of the invention, the composition is administered to the subject parenterally intravenously, subcutaneously, topically, intradermally, orally, sublingually, intranasally or intramuscularly.

The therapeutic use of the invention may be also formulated as:
- Use the composition of the invention, the pharmaceutical composition or the nutraceutical composition comprising the composition of the invention, for the manufacture of a composition for hepatoprotection, the treatment and/or prevention of a hepatic disease, or treatment and/or prevention of a disease or condition associated with inflammation in a subject; or
- Method for hepatoprotection, treating and/or preventing a hepatic disease, or treating and/or preventing a disease or condition associated with inflammation in a subject, wherein said method comprises administering the composition of the invention, the pharmaceutical composition or the nutraceutical composition comprising the composition of the invention to the subject.

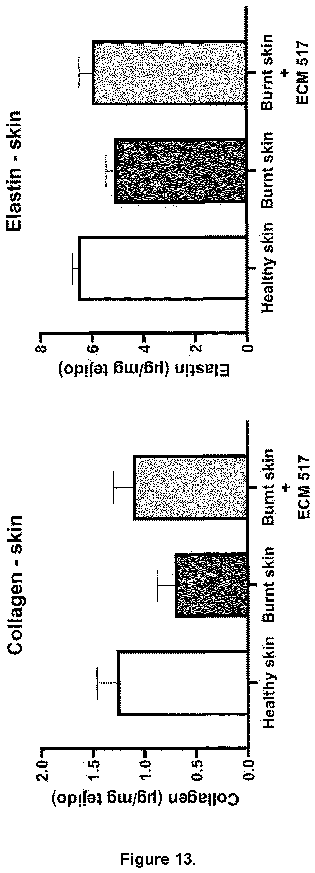

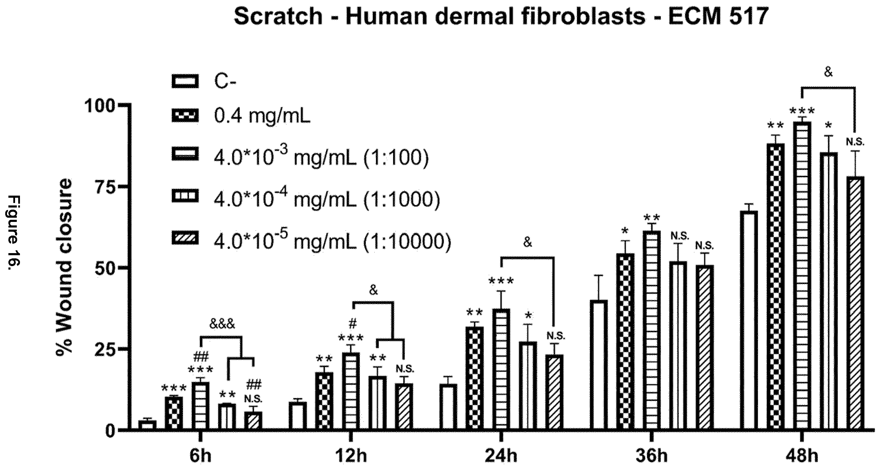

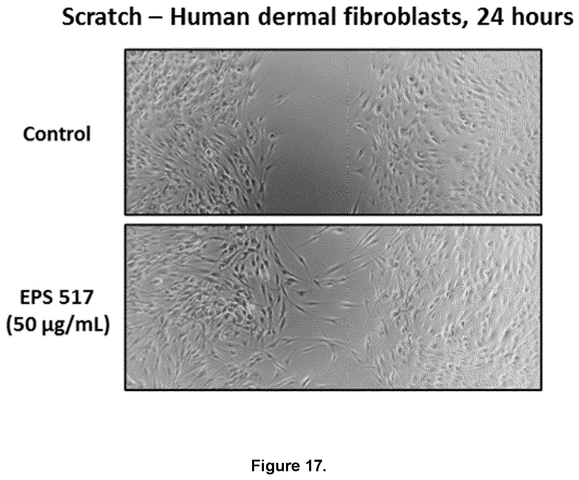

Surprisingly, the extracellular products produced by the strain of the invention showed an anti-inflammatory effect and an increase in the production of collagen and elastin in organotypic cultures of human skin explants, which were subjected to a thermal burn (Figure 13). Additionally, the inventors observed an increase in the wound closure determined by using a scratch assay with human dermal fibroblasts (Figure 16 and Figure 17).

Thus, in another aspect, the present invention relates to the composition of the invention or the pharmaceutical composition comprising the composition of the invention, for use in the treatment of wounds, cuts, thermal burns, chemical burns or abrasions in the skin of a subject, hereinafter the "skin regenerative use of the invention".

In a preferred embodiment of the skin regenerative use of the invention, the composition of the invention is administered, preferably comprised in pharmaceutical composition, at a regenerative effective amount. As used herein, the term "regenerative effective amount" any amount of a compound, agent, mixture or composition that is sufficient to fulfil its skin regenerative intended purpose (e.g. an amount that promotes the wound healing in the skin).

In a preferred embodiment of the skin regenerative use of the invention, the regenerative effective amount of the composition of the invention with regard to the total weight of the pharmaceutical composition, preferably the pharmaceutical composition of the invention, is between 0.0001% (in weight) and 20% (in weight); preferably between 0.0001% (in weight) and 10% (in weight); more preferably between 0.0001% (in weight) and 5% (in weight).

In a preferred embodiment of the skin regenerative use of the invention, the composition is topically administered to the subject.

The skin regenerative use of the invention may be also formulated as:
- Use the composition of the invention, the pharmaceutical composition or the nutraceutical composition comprising the composition of the invention, for the manufacture of a composition for the treatment of wounds, cuts, thermal burns, chemical burns or abrasions in the skin of a subject; or
- Method for treating wounds, cuts, thermal burns, chemical burns or abrasions in the skin of a subject, wherein said method comprises administering the composition of the invention, the pharmaceutical composition or the nutraceutical composition comprising the composition of the invention to the subject.

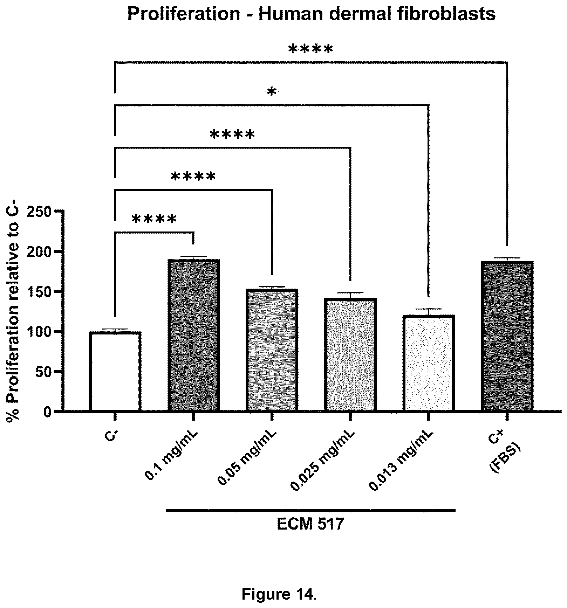

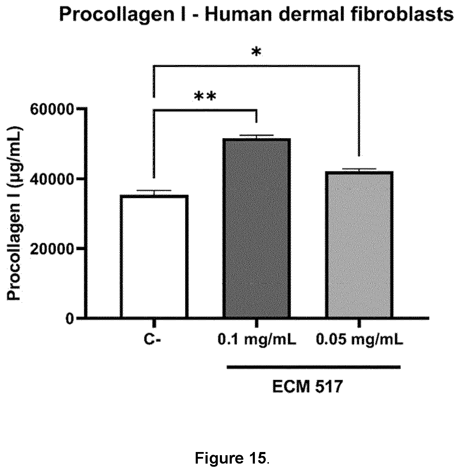

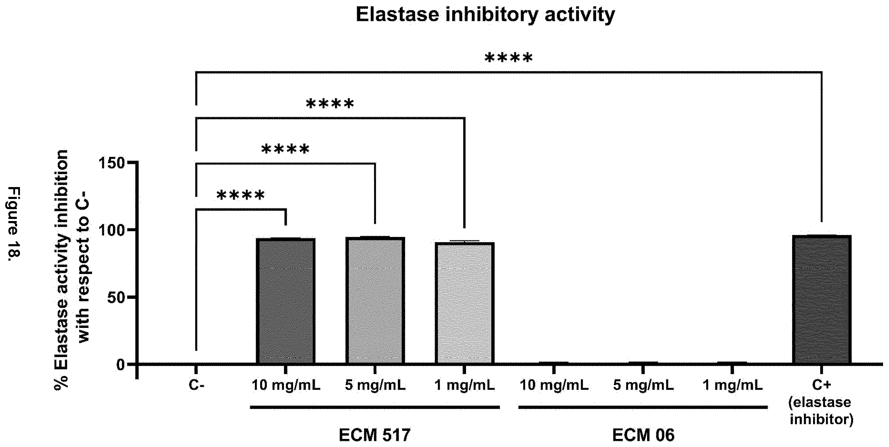

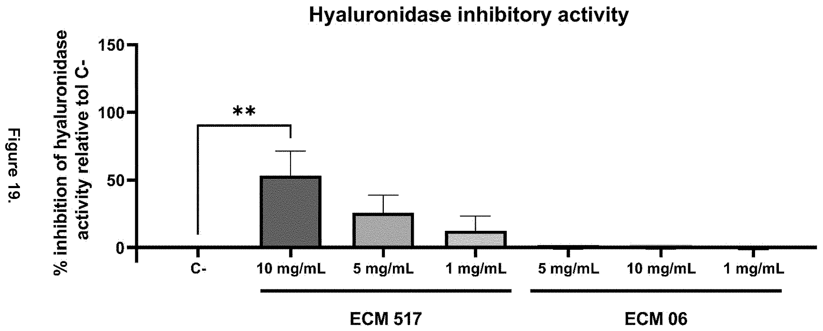

The extracellular products produced by the strain of the invention inhibited the elastase and hyaluronidase enzymes (Figure 18 and Figure 19), increased the production of elastase and procollagen I, and increase the proliferation of human dermal fibroblast (Figure 14 and 15).

Thus, in another aspect, the invention relates to the non-therapeutic cosmetic use of the composition of the invention or the cosmetic composition comprising thereof, for the skin care in a subject, hereinafter "the cosmetic use of the invention".

The term "skin care" refers to the preservation of skin integrity and/or enhancement of its appearance.

In a preferred embodiment the skin care comprises the stimulation of collagen synthesis, promotion of elastin synthesis, prevention of skin aging, prevention of skin wrinkles, improving skin firmness, prevention of loss of skin firmness, and/or improving skin elasticity.

In a preferred embodiment of the cosmetic use of the invention, the composition of the invention is administered, preferably comprised in cosmetic composition, at a cosmetically effective amount. As used herein, the term "cosmetically effective amount" refers to any amount of a compound, agent, mixture or composition that is sufficient to fulfil its non-therapeutic cosmetic purpose (e.g. an amount that produces the preservation of skin integrity and/or enhancement of its appearance).

In a preferred embodiment of the cosmetic use of the invention, the cosmetically effective amount of the composition of the invention with regard to the total weight of the cosmetic composition, is between 0.0001% (in weight) and 20% (in weight); preferably between 0.0001% (in weight) and 10% (in weight); more preferably between 0.0001% (in weight) and 5% (in weight).

In some preferred embodiments of the skin regenerative use of the invention and the cosmetic use of the invention, the composition of the invention or the cosmetic composition comprising thereof, is topically administered to the subject.

In some preferred embodiments, the subject of the therapeutic use of the invention, the skin regenerative use of the invention and the cosmetic use of the invention is selected from a non-human animal or a human being, preferably a human being.

The cosmetic use of the invention may be also formulated as:
- Use the composition of the invention, the pharmaceutical composition or the nutraceutical composition comprising the composition of the invention, for the manufacture of a composition for the skin care in a subject; or
- Method for skin care in a subject, wherein said method comprises administering the composition of the invention, the pharmaceutical composition or the nutraceutical composition comprising the composition of the invention to the subject.

In another preferred embodiment, the composition of the invention presents emulsifying, anti-inflammatory, hepatoprotective and/or wound healing activity, wherein the aforementioned activities can be determined as in the examples of the present application.

### Example 1. Isolation of the strain Pseudoalteromonas sp.517.

*Pseudoalteromonas* sp. 517 was isolated from a seawater sample collected in the Bay of Biscay (44.15°N -2.45°W) at a depth of 500m, by seeding and isolation in marine agar (Difco). The strain *Pseudoalteromonas* sp. 517 was deposited at the at the "Colección Española de Cultivos Tipo, CECT" (Spanish Type Culture Collection) with the address: Parc Cientific de la Universitat de València, Carrer del Catedràtic Agustin Escardino Benlloch, 9, 46980 Paterna, Valencia, Spain; the depositor being Fundación AZTI-AZTI Fundazioa with the address Txatxarramendi ugartea s/n, 48395 Sukarrieta (Vizcaya), Spain. The strain strain *Pseudoalteromonas* sp. 517 was received on 24^{th} February 2023 at the CECT for patent deposit according to the Budapest Treaty, and corresponds to the strain *Pseudoalteromonas sp.* with the deposit number CECT 30790.

### Example 2. Identification of the strain Pseudoalteromonas sp.517.

Amplification of full-length 16S rDNA of the isolate was performed using the universal primers 27F and 1492R as in Delong E. (1992) Proceedings of the National Academy of Sciences, vol.89, issue. 12, pp.5685-5689*.* Taxonomic assignment was done by performing BLASTn against the non-redundant nucleotide sequence and the 16S ribosomal RNA sequences (Bacteria and Archaea) databases from NCBI (https://blast.ncbi.nlm.nih.gov/Blast.cgi).

### Example 3. Obtention of the extracellular products of Pseudoalteromonas sp.517.

To obtain the extracellular matrix of the *Pseudoalteromonas* sp.517 (ECM517, the extracellular matrix isolated from the strain of the invention), the strain was cultivated in MM10 medium with a composition of salts per liter: Na₂HPO₄ (5 g), KH₂PO₄ (2 g), NaCl (30 g), NH₄Cl (7 g), MgSO₄·7 H₂O (0.018 g), CaCl₂ (0.05 g), glucose (20 g), peptone (5 g) and yeast extract (1 g). Cultures were incubated at 10 °C for 3 days under agitation. For ECM517 recovery, bacteria were centrifuged at 10.000 g for 30 min and the clarified supernatants were reserved. Cell pellets were washed twice with NaCl 3% solution and centrifuged again at 18.000×*g* for 40 min. The supernatant was pooled with the supernatant reserved from the broth culture and both were filtered through 0.45 µm. The resulting clear solution was concentrated using a 10 kDa tangential flow cartridge (Sartorius) against distilled water. The concentrated product was filtered through a 0.22 µm filter.

### Example 4. Monosaccharide composition of the EPS produced by Pseudoalteromonas sp.517.

The monosaccharide content of the polysaccharide fraction (exopolysaccharide, EPS) from ECM517 was characterized by using the method based on the formation of per-O-acetyl methyl glycosides. The samples were treated with 1.25 M HCl/MeOH at 85 °C for 16 h. Then, the methyl glycosides were *N*- and *O*-acetylated with a mixture of pyridine and acetic anhydride. The mixture was analysed by gas chromatography-mass spectrometry (GC-MS) using an Agilent HP-5MS gas column (5% Phenylmethylsiloxane, 30 m × 250 µm × 0.25 µm) (C. de Castro et al., Methods Enzymology 480 (2010) 89-115). For identification, the chromatograms were processed using the AMDIS program applying a database created from standards treated under the same conditions. For quantification, calibration curves were created from mixtures of standards of D-galacturonic acid, D-mannose, D-galactose, D-glucose, *N*-acetyl-D-galactosamine and *N*-acetyl-D-glucosamine from 20 to 67 mg/L of each monosaccharide, using the area relative to the internal standard *myo*-inositol (1.33 mg/L). For quantifying the monosaccharides that could not be identified (i.e., heptoses and *N*-acetylated deoxy amino sugars which are not *N*-acetylglucosamine nor *N*-acetylgalactosamine) estimates were obtained based on the response range in the calibration curves from the available standards.

The most abundant monosaccharides were galacturonic acid (13 - 18% by weight), the amino sugar *N*-acetylgalactosamine (7 - 11% by weight), *N*-acetylglucosamine (3-6% by weight) and mannose (4-6% by weight), and (Table 1). As compared to other exopolysaccharides described for *Pseudoalteromonas* genus, the exopolysaccharide from ECM517 is enriched in galacturonic acid (13 - 18% by weight, Table 1) and *N-*acetylgalactosamine (7 - 11% by weight), and it does not present detectable glucuronic acid. The % of each monosaccharide of the ECM517 of *Pseudoalteromonas* sp.517 is calculated with respect to the total weight of the ECM517.

**Table 1. Monosaccharide composition of the polysaccharide fraction from ECM517 matrix, % expressed with respect to the total weight of the ECM517.**

| **Monosaccharide** | **Percentage** |
|---|---|
| ***Galacturonic acid*** | 13 - 18% by weight |
| ***N-Acetylgalactosamine*** | 7 - 11 % by weight |
| ***N-Acetylglucosamine*** | 3 - 6% by weight |
| ***Mannose*** | 4-6 % by weight |
| **Galactose** | 2-4 % by weight |
| ***N-Acetylated deoxy amino sugars (which are not N-acetylglucosamine nor N-acetylgalactosamine)*** | 1-7% by weight |
| ***Heptose*** | 1-6 % by weight |
| ***Glucose*** | 1-2 % by weight |

### Example 5. Imaging of ECM produced by Pseudoalteromonas sp.517.

The observation of the ECM of *Pseudoalteromonas* 517 by transmission electron microscopy (TEM) from a negative stained sample showed the presence of membranous spherical structures with a diameter comprised between 20 and 200nm, that corresponded to membrane vesicles (MVs) (Figure 1).

### Example 6. ¹H-NMR analysis of ECM517.

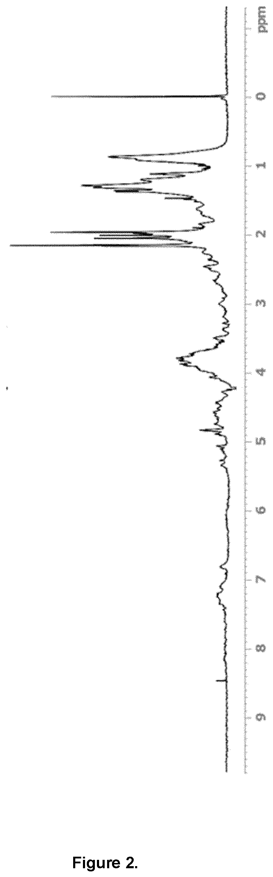

For the preparation of the sample for its analysis by ¹H-NMR the samples were deuterium-exchanged, dissolved in D₂O and their ¹H-NMR spectra were registered on a Bruker Avance 500 NMR spectrometer operating at 500 MHz and 353 K, using deuterated (3-(trimethylsilyl)-2,2,3,3-tetradeuteropropionic acid, TSP) as a chemical shift reference. The analysis confirmed the presence of the polysaccharide fraction in ECM517 by means of identifying the signal in the anomeric zone (approx. 4.5 to 5.5 ppm) and the ring signal zone (about 3.0 to 4.5 ppm, Figure 2). Intense signals were also detected in the area corresponding to acetyl groups (approx. 2.0 ppm) as well as intense and broad signals below 1.5 ppm that correspond to the methylene and methyl groups of fatty acids, components from the membrane vesicles visualized by TEM in ECM517 (Figure 1).

### Example 7. Protein and DNA content of the ECM517.

The amount of DNA and protein associated with ECM517 was quantified by fluorimetry (Quibit Protein assay and Quibit-HS DNA assay), obtaining values of 3.27% ± 0.19 by weight for protein and 0.56% ± 0.11 by weight for DNA. Sodium dodecyl sulfate-polyacrylamide gel electrophoresis (SDS-PAGE) was carried out to determine the molecular weight from ECM517 proteins. Enriched bands were observed in the fraction between 130 and 95 kDa. Other enriched bands were observed with smaller molecular size, mainly at 55, 27 and 17 kDa. The protein pattern of the ECM517 compound is distinct and novel, as it differs considerably from what was previously described (Figure 3).

### Example 8. Technological properties of the ECM517 to identify its potential as food ingredient.

In order to study the **surface properties** of the ECM517 sample (2.6 mg/mL), which are indicative of the capacity of a product to create and stabilize foams and emulsions, interfacial rheology was applied. For this purpose, time sweep measurements were carried out by applying a strain of 0.1 % and at a frequency of 1rad/s for 20 hours at a temperature of 6°C using a rheometer (TA instruments). As a reference, a commercial product of similar characteristics which is used both in food and cosmetics was analyzed: arabic gum. The behaviour of both products (ECM517 and arabic gum) allowed predicting the ability of the ingredients to create and stabilize structures such as emulsions and foams.

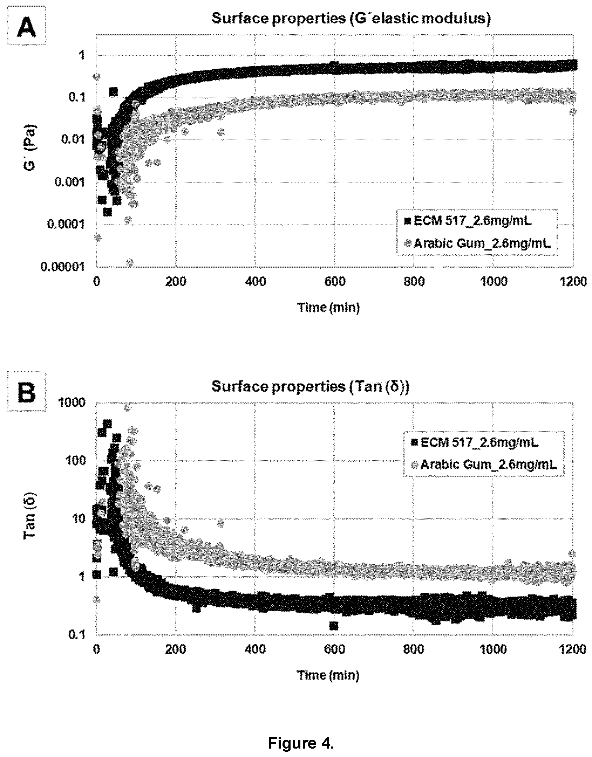

Throughout the measurements, the value of G' (elastic modulus) related to the strength of the air/water interface, increased more markedly in the case of ECM517 with respect to the commercial arabic gum sample. This fact would indicate that ECM517 presents more marked surface properties and therefore could generate more stable structures (foams and emulsions, Figure 4A).

As shown in Figure 4B, the tan (delta) value decreased with time indicating that the elastic behaviour of the interface was improved during adsorption of the compounds to the interface for both samples. In the case of the ECM517 sample, this value was less than 1, indicating a predominantly elastic behavior at the interface for this sample.

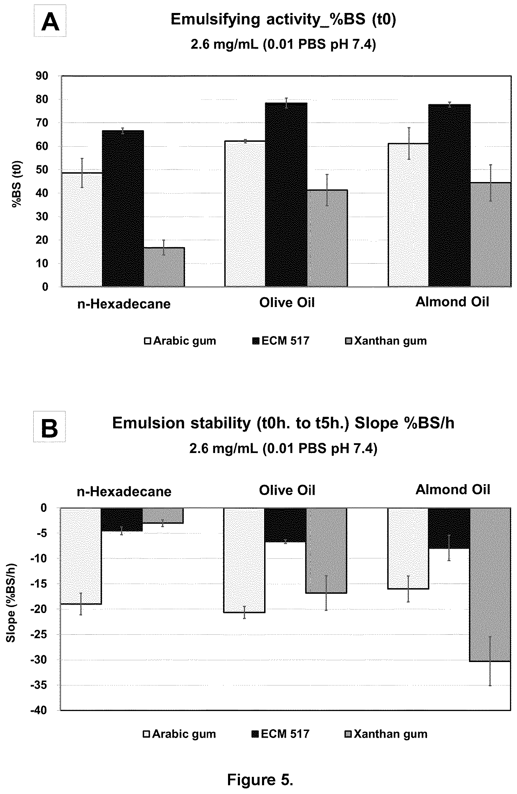

Additionally, the ECM517 and two other reference products, the xanthan gum and the arabic gum, were integrated into emulsions of equal concentration (2.6 mg/mL) at neutral conditions (0.01M of PBS pH7.4) to evaluate their stability over time (20h). For this purpose, the Turbiscan Labexpert (Formulation) equipment was used. A total of 4.5 ml of each solution at concentration 2.6 mg/mL of ECM517, xanthan gum or arabic gum were mixed with 4.5 mL of n-hexadecane (Sigma-Aldrich), olive oil, or almond oil. The emulsions were prepared using a homogenizer POLYTRON^{®} PT 10-35 GT at 15.000 rpm for 2 min and were immediately measured to obtain the % of BS (back scattering) at time 0 min. This value allows identifying the emulsifying activity of a sample. Higher % of BS is related to higher emulsifying activity. As shown in Figure 5A, the **emulsifying activity** of the ECM517 was higher than that obtained from the xanthan gum sample and the arabic gum sample.

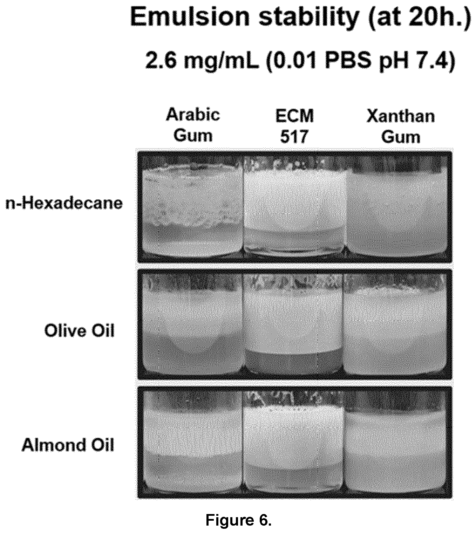

To analyse the emulsion stability, the evolution of the % of BS was measured during a period of 20 hours at room temperature. Marked changes in the % of BS (%BS/h, slope time 0h to time 5h) would indicate lower stability of the emulsions (Figure 5B). The analysis of the emulsifying properties clearly shows that, under the conditions used, ECM517 generated more stable emulsions compared to the arabic gum and the xanthan gum (Figures 5B and 6).

In addition, the **viscosity** at neutral conditions (0.01M of PBS pH7.4) of the ECM517, and arabic gum samples (3.28 mg/mL) were measured using a rheometer (TA instruments). For this purpose, flow sweep measurements were carried out by applying a shear rate from 0.001 s-1 to 100 s-1 at room temperature using a concentric cylinder geometry. The viscosity analysis showed that the EPS of the invention, or the ECM of the invention, generated solutions with higher viscosity compared to arabic gum (Figure 7).

### Example 9. Nutraceutic activity of the ECM517.

Different in vitro assays with human cell lines were performed with the objective of analysing the bioactivity of the ECM517 in the nutraceutic field. The **anti-inflammatory activity** was determined by quantifying the secretion of pro- and inflammatory cytokines (TNF-α and IL-8) by a culture of macrophages exposed to an inflammatory stimulus (LPS, 10 µg/mL) in the presence and absence of ECM517 at different concentrations (0.1, 0.05, 0.025 and 0.0125 mg/mL). The ECM517 showed statistically significant anti-inflammatory activity when applied at the concentration of 0.1 mg/mL (Figure 8).

The study of the **hepatoprotective activity** was performed by quantifying the cell viability of human hepatocytes (HepG2) exposed to the hepatotoxic drug methotrexate (MTX), in the presence and absence of different concentrations of ECM517 (0.5 mg/mL and 0.1 mg/mL). The results obtained reveal a statistically significant hepatoprotective effect of ECM517 at all concentrations tested (0.5 mg/mL and 0.1 mg/mL, Figure 9).

To determine whether ECM517 exhibits **proliferative activity in the gastrointestinal context,** a cell proliferation study was performed in the human gastrointestinal cell line, Caco-2. For this purpose, the percentage of cell proliferation in the presence of different concentrations of ECM517 (0.5 mg/mL, 0.1 mg/mL, 0.05 mg/mL and 0.01 mg/mL) versus the negative control (i.e., untreated cells) was studied. Both the negative control and the experimental groups under study were in nutrient-depleted medium. In parallel, a positive control experimental group was also included, which included an extra nutrient supply (FBS, fetal bovine serum). ECM517 stimulated cell proliferation above the basal levels of the negative control with statistical significance at the following concentrations: 0.5 mg/mL, 0.1 mg/mL, 0.05 mg/mL and 0.01 mg/mL. The values obtained show an increase in cell proliferation of ca. 20%, reaching values close to the positive control (Figure 10). Similar results are represented in Figure 11 which corresponds to a wound closure study (scratch assay) in which microscopy photographs were taken after 24 hours of exposure to ECM517.

### Example 10. Dermocosmetic and therapeutic activity of ECM517.

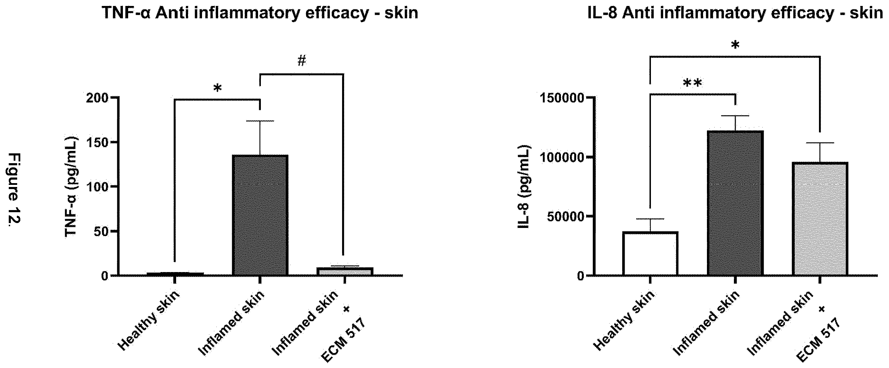

In addition to demonstrating the anti-inflammatory activity of the ECM517 in the eukaryotic model (Figure 8), an **anti-inflammatory study was carried out in organotypic cultures of human skin, which were subjected to a thermal burn.** The inflammatory state (secretion of pro- and inflammatory cytokines, IL-8 and TNF-α) of these explants subjected to the burn with and without treatment with ECM517 at 0.1 mg/mL concentration was analyzed. The results are shown in Figure 12. The compound ECM517 shows anti-inflammatory activity in human skin explants where inflammation (thermal burn) had been produced at the concentration of 0.1 mg/mL.

The **regenerative activity** for ECM517 was studied by determining the levels of collagen and elastin secreted in the skin model which had been subjected to thermal burn with subsequent application of ECM517. An increase in both collagen and elastin levels was observed after thermal burn generation in human skin explants in response to ECM517 exposure illustrating its regenerative activity. These levels approximated the levels of the healthy skin control. The results obtained are presented in Figure 13.

The **proliferative activity of ECM517 in human dermal fibroblasts** was studied after 72 hours of exposure in a serum-deprived medium. The results show that ECM517, at all concentrations tested (0.1 mg/mL, 0.05 mg/mL, 0.025 mg/mL and 0.013 mg/mL), induces proliferation of human dermal fibroblasts (Figure 14) compared to the negative control (cells that were not exposed to any treatment).

In addition to studying proliferation in fibroblasts and quantifying collagen in the skin after exposure to ECM517, **collagen secretion was studied in cultured human dermal fibroblasts** at two concentrations of ECM517 (0.1 mg/mL and 0.05 mg/mL). The results (Figure 15) show an induction in the secretion of procollagen I (collagen precursor) in this cell type, thus evidencing the reparative activity of ECM517 at these concentrations.

Additionally, the ***in vitro* healing activity** was studied using the scratch assay in human dermal fibroblasts (HDF), in the presence and absence of different concentrations of ECM517 (0.4 mg/mL, 0.004 mg/mL, 0.0004 mg/mL and 0.00004 mg/mL). The results are depicted in Figure 16, which shows a significant increase in wound closure at concentrations ranging from 0.0004 mg/mL to 0.4 mg/mL. Additionally, a microscopy image of the dermal fibroblast culture is shown, in which the "wound healing" effect favored by the presence of ECM517 is observed (Figure 16).

### Inhibition of elastase action

In humans, unregulated protease activity is associated with various pathological conditions such as cancer, inflammatory processes, thrombosis, arthritis, skin diseases and pulmonary emphysema. In particular, elastase protease action is associated with a number of diseases, such as liver failure, rheumatoid arthritis, psoriasis, skin cancer, arteriosclerosis and various pulmonary pathologies (Johansson et al., 2002, J. Nat. Prod.65,32-41). In this context, the possible activity of ECM517 as an inhibitor of elastase activity was evaluated. An ECM obtained from another *Pseudoalteromonas* strain *(Pseudoalteromonas* sp. 06, ECM06) isolated from the same environment as *Pseudoalteromonas* sp. 517 was also included for comparison. For this purpose, elastase activity was measured in the presence and absence of ECM517 and ECM06 at different concentrations, in parallel, an enzyme activity inhibitor widely described in the literature was also included as a positive control, C+ (N-(methoxysuccinyl)-L-alanyl-L-alanyl-L-proyl-L-valine chloromethylketone, 0.005 mg/mL). At all the concentrations tested (10 mg/mL, 5 mg/mL and 1 mg/mL), EMC517 shows elastase inhibitory activity, reaching levels similar to those of the positive control, while EMC06 did not show activity (Figure 17).

### Hyaluronidase Inhibitor

In recent years, hyaluronidases have received much attention because of their ability to abruptly alter hyaluronic acid homeostasis. These enzymes preferentially degrade hyaluronic acid, a structural polysaccharide found exclusively in the extracellular matrix of animals and humans. The hyaluronic acid-hyaluronidase system is known to be involved in many pathophysiological conditions. In this context, hyaluronidase inhibitors prove to be potent regulators that maintain hyaluronic acid homeostasis and could serve as anti-inflammatory, anti-aging, antimicrobial, anticancer, and antivenom/toxin and contraceptive agents. We aimed at evaluating the ability of ECM517 at different concentrations on hyaluronidase activity. An ECM obtained from another *Pseudoalteromonas* strain isolated from the same environment (ECM06) was also included as a comparison. An inhibition of hyaluronidase activity dependent on the concentration of ECM517 present in the assay was observed, while ECM06 did not show activity at any of the concentrations tested (Figure 19).

### DESCRIPTION OF FIGURES

**Figure 1****.** Image of compound ECM517 obtained by transmission electron microscopy (TEM) of negative stained samples. Membrane vesicles (MVs) can be observed with membranous bilayers. Bar, 200 nm.
**Figure 2****.** ¹H-NMR spectra (500 MHz, 353 K) of the compound ECM517.
**Figure 3****.** Protein profile of ECM517 using 12.5% Dodecyl sulfate polyacrylamide gel electrophoresis (SDS-PAGE). Numbers on the left correspond to molecular weight (MW) in thousands. Enriched bands were observed in the fraction between 130 and 95 kDa and other enriched bands were observed with smaller molecular size, mainly at 55, 27 and 17 kDa.
**Figure 4****.** Evolution over time of the elastic modulus (Figure 4A) and the delta tangent (Figure 4B) at the air/water adsorption interface of the ECM517 and the arabic gum.
**Figure 5****.** Emulsifying activity (A) and emulsion stability (B) over 5 hours of the emulsions made with ECM517, xanthan gum and arabic gum measured at room temperature. A higher %BS indicates higher emulsifying activity. A higher %BS/h change (slope with marked negative values), indicates higher destabilization.
**Figure 6****.** Stability of the emulsions made with ECM517, xanthan gum and arabic gum mixed with different food and cosmetic oils and n-hexadecane after 20 hours storage at room temperature.
**Figure 7****.** Analysis of the viscosity (mPa.s) of the ECM517 and arabic gum solution, measured at 25°C.
**Figure 8****.** Graphical representation of TNF-α and IL-8 secretion by human macrophages stimulated with LPS in the presence of ECM517. Control - indicates the basal values of the non-inflamed culture. LPS control - the values of the inflamed culture, exposed to LPS. ***: p < 0.005 vs C+ LPS. ****: p < 0.001 vs. C+ LPS.
**Figure 9****.** Graphical representation of cellular hepatoprotection of ECM517 in the human cell line, HepG2 treated with a hepatotoxic agent (methotrexate, MTX). Asterisks indicate ****: p < 0.001 vs. C+ MTX.
**Figure 10****.** Graphical representation of cell proliferation in the human intestinal cell line, Caco-2 in the presence of ECM517, relative to the negative control. The positive control experimental group contains the nutrient supply provided by FBS. Asterisks indicate **: p < 0.01 vs C-. ***: p < 0.005 vs C-. ****: p < 0.001 vs C-.
**Figure 11****.** Microscopy image of the scratch assay of Caco-2 culture exposed to ECM517. The Caco-2 cell culture was exposed to ECM517 at 50µg/mL for 24 hours. After that time wound closure was visually assessed by microscopy.
**Figure 12****.** Graphical representation of the anti-inflammatory activity (IL-8 and TNF-α secretion) of ECM517 in human skin explants. Control - (healthy skin), basal values of the non-inflamed culture. Positive Control, (inflamed skin). *p<0.05 relative to healthy skin. **p<0.01 with respect to healthy skin. # p<0.05 with respect to inflamed skin.
**Figure 13****.** Regenerative activity of ECM517 in skin. Collagen (left) and elastin (right) levels after application of ECM517 in a skin model with thermal burn damage.
**Figure 14****.** Graphical representation of dermal fibroblast proliferation in the presence of ECM517. Asterisks indicate *: p < 0.05 vs C-. ***: p < 0.005 vs C-. ****: p < 0.001 vs C-.
**Figure 15****.** Graphical representation of procollagen I secretion in human dermal fibroblasts after 72-hour exposure to ECM517. Asterisks indicate *: p < 0.05 vs C-. **: p < 0.01 vs C-.
**Figure 16****.** Graphical representation of the scratch assay in human dermal fibroblasts (HDFs). Asterisks indicate: *: p < 0.05 vs Ctrl. **: p < 0.005 vs Ctrl. ***: p < 0.001 vs Ctrl. #: p < 0.05 vs 0.4 mg/mL. ##: p < 0.005 vs. 0.4 mg/mL. &: p < 0.05 between groups. &&&: p < 0.05 between groups. N.S. not significant vs. control.
**Figure 17****.** Microscopy of the scratch assay of dermal fibroblast culture exposed to ECM517. The fibroblast cell culture was exposed to ECM517 at 50µg/mL for 24 hours. After that time, wound closure was visually assessed by microscopy.
**Figure 18****.** Elastase inhibitory activity of ECM517 and ECM06 at different concentrations. Asterisks indicate ****: p < 0.001 vs C-.
**Figure 19****.** Hyaluronidase inhibitory activity of ECM517 and ECM06 at different concentrations. Asterisks indicate **: p < 0.01 vs C-.

## Claims

1. A bacterial strain *Pseudoalteromonas* sp. 517 with the deposit number CECT 30790.

2. Method for the obtention of a composition comprising an exopolysaccharide, comprising the following steps:
a) culturing bacterial strain according to claim 1; and
b) isolating the composition comprising the exopolysaccharide from the secreted fraction of the culture.

3. A composition comprising an exopolysaccharide, obtainable by the method according to claim 2, wherein the exopolysaccharide comprises galacturonic acid, N-acetylgalactosamine, mannose, N-acetylglucosamine, galactose, glucose, *N-*acetylated deoxy amino sugars that are not *N*-acetylglucosamine nor *N-*acetylgalactosamine and at least one heptose.

4. The composition according to claim 3, wherein the exopolysaccharide comprises less than 5 % by weight of glucose respect to the total weight of the composition.

5. The composition according to claim 3 or 4, wherein the exopolysaccharide comprises from 10 % to 20% by weight of galacturonic acid, from 5% to 12% by weight of N-acetylgalactosamine, from 4 to 7% by weight of mannose, from 2 % to 8 % by weight of N-acetyl glucosamine, from 2 % to 5% by weight of galactose and 1 % to 4% by weight of glucose, from 1 to 8% by weight of N-acetylated deoxy amino sugars which are not N-acetylglucosamine nor N-acetylgalactosamine and from 1% to 6% by weight of at least one heptose, wherein the percentages are expressed respect to the total weight of the composition.

6. The composition according to any one of claims 3 to 5, further comprising extracellular vesicles.

7. The composition according to any one of claims 3 to 6, further comprising proteins.

8. The composition according to any one of claims 3 to 7, wherein said composition is an extracellular matrix (ECM).

9. A pharmaceutical, nutraceutical, cosmetic or food composition that comprises the composition according to any one of claims 3 to 8.

10. Use of the composition according to any one of claims 3 to 8, as emulsifying, thickening and/or stabilizing agent.

11. Use of the composition according to any one of claims 3 to 8, as food ingredient for the manufacture of food products.

12. The composition according to any one of claims 3 to 8, the pharmaceutical composition according to claim 9 or the nutraceutical composition according to claim 9, for use as a medicament.

13. The composition according to any one of claims 3 to 8, the pharmaceutical composition according to claim 9 or the nutraceutical composition according to claim 9, for use in hepatoprotection, in the treatment and/or prevention of a hepatic disease, or in the treatment and/or prevention of a disease or condition associated with inflammation in a subject; wherein the hepatic disease is selected from the group consisting of liver transplantation, alcoholic or non-alcoholic fatty liver, hepatic fibrosis, hepatocirrhosis and hepatitis caused by virus or drug, alcoholic acute/chronic hepatic disease, fatty acid-induced fatty liver or acute/chronic hepatic disease derived from fatty liver or a hepatic disease is mediated by reactive oxygen species (ROS) or heavy metal; and wherein the disease or condition is selected from the list consisting of the group consisting of allergy, cardiovascular disease, neurologic disease, pulmonary disease, cancer, metabolic disease, auto-immune disease, gastrointestinal disease, and skin or mucosal disease or disorder.

14. The composition according to any one of claims 3 to 8, or the pharmaceutical composition according to claim 9, for use in the treatment of wounds, ulcers, cuts, thermal burns, chemical burns or abrasions in the skin of a subject.

15. Non-therapeutic cosmetic use of the composition according to any one of claims 3 to 8 or the cosmetic composition according to claim 9, for the skin care in a subject.
